Europäisches Patentamt

European Patent Office

Office européen des brevets

(19)

(11) Publication number: **0 093 438**
**B1**

(12) **EUROPEAN PATENT SPECIFICATION**

(45) Date of publication of patent specification: **20.07.88**

(21) Application number: **83104249.4**

(22) Date of filing: **30.04.83**

(51) Int. Cl.⁴: **A 61 K 39/29**

(54) **Non-A, Non-B hepatitis antigen.**

(30) Priority: **05.05.82 US 374846**

(43) Date of publication of application:
**09.11.83 Bulletin 83/45**

(45) Publication of the grant of the patent:
**20.07.88 Bulletin 88/29**

(84) Designated Contracting States:
**DE FR GB**

(56) References cited:
**EP-A-0 061 974**
**EP-A-0 066 296**
**WO-A-80/02598**

**Lancet October 21, 1978 pp. 853-56**

(73) Proprietor: **The Board of Supervisors of Louisiana State University and Agricultural and Mechanical College**
-
**Baton Rouge, Louisiana 70803 (US)**

(72) Inventor: **Villarejas, Victor M., Dr.**
**19 Bello Horizonte**
**Escazu, San Jose (CR)**
Inventor: **Visona, Kirsten A., Dr.**
**Los Colegios**
**Moravia, San Jose (CR)**

(74) Representative: **UEXKÜLL & STOLBERG**
**Patentanwälte**
**Beselerstrasse 4**
**D-2000 Hamburg 52 (DE)**

EP 0 093 438 B1

## Description

### Background of the Invention

Since the discovery of hepatitis B surface antigen (HBsAg) by Blumberg, Bull. N. Y. Acad. Med. 1964, *40*, 377 and of hepatitis A virus (HAV) Feinstone et al., Science, 1973, *182*, 1026, hepatitis types A and B have become distinguishable by specific serological tests. A further type of hepatitis, in which no agent has yet been identified, has been designated "non-A, non-B hepatitis". In the United States of America, where HBsAg-positive donor blood now is excluded from use in transfusions, non-A, non-B hepatitis accounts for approximately 89% of cases of post-transfusion hepatitis, Alter et al., Lancet 2:838—841, 1975, Alter et al., Am. J. Med. Sci., 270:329—334, 1975. Between 5.4 and 18.5% of Americans receiving five units or less of transfused blood develop non-A, non-B hepatitis, Alter et al., op. cit. Although the name "hepatitis C" has been proposed for this disease, most clinicians and investigators have continued to refer to it as non-A, non-B hepatitis because circumstantial evidence suggests that more than one agent may transmit this disease. The diagnosis of non-A, non-B hepatitis is still based on the exclusion by serologic tests of known etiologic agents: HAV and hepatitis B virus (HBV), cytomegalovirus, Epstein-Barr virus, varicellazoster, or herpes simplex.

In "The Lancet" of October 21 in 1987 pages 853 to 856 R. Shirachi et al. described an antigenic substance with a density of 1.3 and a molecular weight of 100 000 to 300 000 which they called Hepatitis "C"-antigen and which was detected in the sera of post transfusion hepatitis patients negative for $HB_sAg$ and $HB_s$-antibody. The substance was primarily observed in sera of patients suffering from type-2 hepatitis characterized by biphasic values of the serum-glutamic-pyruvic-transaminase. However, antibodies against the described antigen were only observed in very few cases (30% of all cases examined) and correlation between infectivity and antigenicity of the antigen has not been confirmed.

### Objects of the Invention

It is, accordingly, an object of the present invention to provide a method for the isolation of non-A, non-B hepatitis antigen. Another object is to provide a method for the detection of non-A, non-B antigen. A further object is to provide non-A, non-B hepatitis antigen which has immunogenic efficacy and may be used as a vaccine against non-A, non-B hepatitis. Still another object is to provide a composition containing non-A, non-B hepatitis which has utility as a diagnostic agent. These and other objects of the present invention will be apparent from the following description.

### Summary of the Invention

Non-A, non-B hepatitis antigen having a particle size of from about 2.0 to about 5.0 nm and a density of from 1.24 to 1.29 g/ml in CsCl.

The foregoing antigen is isolated by isopycnic banding and chromatographic fractionation.

### Detailed Description of the Invention

According to the present invention a sample of blood from a patient clinically diagnosed as having non-A, non-B diagnosis by

1) elevated liver enzymes determined by specific assays used in diagnosing liver disease,

2) possible clinical symptoms including jaundice, and

3) absence of HAV or HBV current active infection as determined by diagnostic assays is converted into serum by standard methods. The serum is then used to screen sera from blood donors to locate serum containing non-A, non-B antibody. An immunodiffusion assay is used to determine the presence of antibody in a specific serum sample.

Once serum containing non-A, non-B antigen is identified, the serum is subjected to isopycnic banding and fractions of the density gradient are assayed for antigenic activity using the antibody previously located using the immunodiffusion assay. The density gradient may be, for example, CsCl, NaBr, or sucrose. In the case of CsCl the non-A, non-B hepatitis antigen is found in the region having a density of from 1.24 to 1.29 g/ml. This antigen has a particle size of from about 2 to about 5 nm and is stable between pH 4 and pH 11. This antigen may be a subunit component of large viral-like particles of up to about 40 nm which have been observed in higher density regions of from about 1.31 to about 1.34 g/ml, and which show the same antigenic characteristics.

The density gradient fractions having antigenic activity are pooled and placed on a chromatographic column. Fractions from the column are analyzed using the immunodiffusion assay using previously described antibody, and fractions showing presence of antigen are collected. Antigenicity is found in those fractions having a molecular weight of from 250,000 to 1,300,000 daltons. Maximum antigenicity is found in those fractions having a molecular weight of from about 400,000 to about 600,000 daltons. Sodium dodecyl sulfate treated antigen shows a component having a molecular weight of about 50,000 daltons using the western block method, Tobin et al., J. Proc. Nat. Acad. Sci. Vol. 76, p. 4350 (1979).

The fractions may be dialyzed if the antigen is desired in a solvent other than that used to elute the column.

The antigen may be inactivated for vaccine use by treatment with formalin at a concentration of from about 1:1,000 to about 1:10,000 for from about 24 to about 120 hours, at any temperature. Preferred conditions are a formalin concentration of about 1:4,000 for about 72 hours at a temperature of from about 33 to about 41°C.

The antigen optionally may be adsorbed on alum to increase its immunogenicity and optionally may be administered in conjunction with other adjuvants.

The non-A, non-B hepatitis antigen of the present invention is immunogenic to susceptible species, e.g., chimpanzees and humans, at a dosage level of from about 1 to about 100 μg, preferably from about 5 to about 50 μg, and most preferably from about 10 to about 30 μg. The following examples illustrate the present invention without, however, limiting the same thereto.

Example 1
Isolation of Non-A, Non-B Hepatitis Antigen

An individual having clinical symptoms of hepatitis disease as determined by elevated levels of serum glutamic oxaloacetic transaminase and serum glutamic pyruvate transaminase enzymes, presence of jaundice, and who was determined not to be suffering from either HAV or HBV by radio-immune diagnostic assays, was bled and the blood converted into serum by known methods. Samples of this serum were used to screen various samples of serum until a serum containing antibody as determined by the immunodiffusion assay (Abbott® rheophoresis plate) was located.

The serum, 7 ml, containing the non-A, non-B antigen was placed on a 31 ml CsCl density gradient made up of five 6.2 ml quantities of the following densities of CsCl: 1.1, 1.2, 1.3, 1.4, and 1.6. The density gradient with the serum sample on top was placed in a Beckman® SW27 rotor and spun at 25,000 rpm for 16 hours. One ml fractions were taken and dialyzed against water. The fractions were then lyophilized and reconstituted to 200 μl with distilled water. The fractions were then assayed for antigenic activity in the immunodiffusion assay using the previously described antibody. Activity was found at a density between 1.24 and 1.29 g/ml with a maximum at 1.27.

A 10 ml Sepharose® CL6B column having a length of approximately 25 cm and a diameter of approximately 7 mm was packed by conventional methods. A charge, 0.4 ml, of pooled density gradient fractions that had been dialyzed and reconstituted as described in the preceding paragraph were placed on the column. The column was eluted with phosphate buffered saline (PBS) at approximately 1 ml per 15 minutes and 0.5 ml fractions were collected. These fractions were then lyophilized and reconstituted in distilled water at 100 μl per fraction. The fractions were then analyzed in the immunodiffusion assay using the previously isolated antibody. The maximum antigenic activity corresponded to those fractions having a molecular weight of approximately 500,000 daltons with activity ranging from 250,000 to 1,300,000 daltons.

Example 2
Preparation of Non-A, Non-B Hepatitis Vaccine

The material eluted from the Sepharose® CL6B column in Example 1 having a molecular weight ranging from 250,000 to 1,300,000 daltons is pooled and treated with formalin at a concentration of 1:4,000 for 72 hours at 37°C to inactivate the antigen and provide a material suitable for use as a vaccine.

Example 3
Preparation of Alum-Adsorbed Vaccine

The inactivated product of Example 2 is adsorbed on alum and suspended in physiological saline solution buffered at from pH 6.0 to pH 7.8.

Example 4

The fractions eluted from the Sepharose® CL—6B in Example 1 showing antigenic activity are dialyzed against distilled water to provide the non-A, non-B antigen in a composition comprising distilled water. This composition is useful either as a vaccine after inactivation as described in Example 2 or as such or as a diagnostic antigen.

Example 5

The fractions eluted from the Sepharose® CL—6B column in Example 1 which show antigenic activity are dialyzed against saline to provide the non-A, non-B antigen in a composition comprising saline. This composition is useful either as a vaccine after inactivation as described in Example 2 or as such or as a diagnostic antigen.

Example 6

Approximately 10 mg of non-A, non-B hepatitis antigen from the Sepharose® CL—6B column was treated with 2% sodium dodecyl sulfate and 5% 2-mercaptoethanol in 5 ml of tris buffer adjusted to pH 6.8 with concentrated HCl. The solution was incubated at 100°C for 3 minutes and 0.5 ml of 50% glycerol and 0.5% bromophenol blue tracking dye were added to the solution. The preparation was applied to a 450 cm³ column of 12.5% polyacrylamide gel with a 5% stacking gel over it. The gel column was developed with 1.5 liters of tris-glycine-sodium dodecyl sulfate buffer in an electric field of 100 volts. Development was continued for 15 hours. A p50,000 daltons molecular weight band was located in the gel by comparison with a coomassie blue stained track run in parallel. The region was cut from the gel and eluted with 20 ml of physiological saline/ 0.1% sodium dodecyl sulfate solution. The sodium dodecyl sulfate solution was removed by extensive dialysis against physiological saline and was ready for formulation into a vaccine.

**Claims**

1. A substantially purified non-A, non-B hepatitis antigen consisting essentially of par-

ticles having a size from about 2.0 to about 5.0 nm, and a density of from 1.24 to 1.29 g/ml in CsCl.

2. The antigen according to Claim 1 having a molecular weight of from 250,000 daltons to 1,300,000 daltons.

3. A subunit antigenic component of the non-A, non-B hepatitis antigen of Claim 1 having a molecular weight when treated with SDS of 50,000 daltons.

4. A composition comprising the antigen of Claim 1 in an aqueous vehicle.

5. A composition according to Claim 4 wherein the vehicle is water, saline or phosphate buffered saline.

6. The antigen according to Claim 1 adsorbed on alum.

7. A vaccine comprising an immunologically effective amount of the antigen of Claim 1 and a physiologically acceptable vehicle.

8. A diagnostic reagent comprising the antigen of Claim 1 in an aqueous medium in a quantity effective to form an immune complex in a diagnostic assay.

9. A process of isolating non-A, non-B hepatitis comprising subjecting serum containing non-A, non-B hepatitis antigen to isopycnic banding and selecting a fraction having a density equivalent to from 1.24 to 1.29 g/ml in CsCl, and a particle size of from 2.0 to 5.0 nm.

10. A process according to Claim 9 wherein the selected fraction is treated to separate particles having a molecular weight of from 250,000 to 1,300,000 daltons.

11. A process according to Claim 9 wherein the treatment separates particles having a molecular weight of from 400,000 to about 600,000 daltons.

## Patentansprüche

1. Im wesentlichen gereinigtes non-A, non-B Hepatitis Antigen in der Hauptsache bestehend aus Partikeln mit einer Größe von etwa 2,0 bis etwa 5,0 nm und einer Dichte von 1,24 bis 1,29 g/ml in CsCl.

2. Antigen nach Anspruch 1 mit einem Molekulargewicht von 250.000 Dalton bis 1.300.000 Dalton.

3. Antigene Komponente als Untereinheit des non-A, non-B Hepatitis Antigens nach Anspruch 1 mit einem Molekulargewicht von 50.000 Dalton nach Behandlung mit SDS.

4. Zusammensetzung enthaltend das Antigen nach Anspruch 1 in einem wäßrigen Träger.

5. Zusammensetzung nach Anspruch 4, dadurch gekennzeichnet, daß der Träger Wasser, Kochsalzlösung oder Phosphat gepufferte Kochsalzlösung ist.

6. Antigen nach Anspruch 1, adsorbiert an Aluminiumsulfat.

7. Vaccine enthaltend eine immunologisch wirksame Menge des Antigens nach Anspruch 1 und einen physiologisch verträglichen Träger.

8. Diagnostisches Mittel enthaltend das Antigen nach Anspruch 1 in einem wäßrigen Medium in einer Menge, die ausreicht, um in einem diagnostischen Nachweisverfahren einen Immunkomplex zu bilden.

9. Verfahren zum Isolieren von non-A, non-B Hepatitis Antigenen, dadurch gekennzeichnet, daß man ein non-A, non-B Hepatitis Antigene enthaltendes Serum einer isopycnischen Banden-Trennung unterwirft und eine Fraktion mit einer Dichte entsprechend 1,24 bis 1,29 g/ml in CsCl und einer Partikelgröße von 2,5 bis 5,0 nm auswählt.

10. Verfahren nach Anspruch 9, dadurch gekennzeichnet, daß man die ausgewählte Fraktion behandelt um Partikel mit einem Molekulargewicht von 250.000 bis 1.300.000 Dalton abzutrennen.

11. Verfahren nach Anspruch 9, dadurch gekennzeichnet, daß man mit der Behandlung Partikel mit einem Molekulargewicht von 400.000 bis etwa 600.000 Dalton abtrennt.

## Revendications

1. Antigène d'hépatite non-A, non-B, pratiquement purifié, comprenant essentiellement des particules ayant une dimension comprise entre environ 2,0 et environ 5,0 nm, et une densité comprise entre 1,24 et 1,29 g/ml dans CsCl.

2. Antigène selon la revendication 1 ayant un poids moléculaire compris entre 250.000 daltons et 1.300.000 daltons.

3. Composant antigénique sous-unité de l'antigène d'hépatite non-A, non-B ayant un poids moléculaire de 50.000 daltons quand on le traite avec du SDS.

4. Composition comprenant l'antigène de la revendication 1 dans un véhicule aqueux.

5. Composition selon la revendication 4, où le véhicule est de l'eau, une solution saline ou une solution saline tamponnée avec du phosphate.

6. Antigène selon la revendication 1 adsorbé sur de l'alun.

7. Vaccin contenant une quantité, efficace immoniquement, de l'antigène de la revendication 1 et un véhicule physiologiquement acceptable.

8. Réactif diagnostique comprenant l'antigène de la revendication 1 dans un milieu aqueux en une quantité efficace pour former un immuncomplexe dans un test diagnostique.

9. Procédé d'isolement de l'antigène d'hépatite non-A, non-B, consistant à soumettre le sérum contenant l'antigène non-A, non-B, à la production de bandes isopycniques et à sélectionner une fraction ayant un équivalent de densité compris entre 1,24 et 1,29 g/ml dans CsCl, et une dimension de particules comprise entre 2,0 et 5,0 nm.

10. Procédé selon la revendication 9, où l'on traite la fraction sélectionnée pour séparer les particules ayant un poids moléculaire compris entre 250.000 et 1.300.000 daltons.

11. Procédé selon la revendication 9, où l'on sépare par le traitement les particules ayant un poids moléculaire compris entre 400.000 et environ 600.000 daltons.